Europäisches Patentamt

European Patent Office

Office européen des brevets

(19)

(11) Publication number:  0 336 523
A1

## (12) EUROPEAN PATENT APPLICATION

(21) Application number: 89200979.6

(22) Date of filing: 06.04.89

(51) Int. Cl.4: **C12N 15/00 , A61K 45/00 , C12P 21/00 , //(C12P21/00, C12R1:91)**

(30) Priority: 08.04.88 EP 88810231

(43) Date of publication of application:
**11.10.89 Bulletin 89/41**

(84) Designated Contracting States:
**AT BE CH DE ES FR GB GR IT LI LU NL SE**

(71) Applicant: **IntraCel Corporation**
**c/o Cottle Catford & Co. 17 High Street**
**Bridgetown(BB)**

(72) Inventor: **Dreano, Michel**
**330, Route de Genève**
**F-74160 Collonge-Sous-Saleve(FR)**
Inventor: **Bromley, Peter**
**26, Chemin de Crêt-de-la-Neige**
**CH-1234 Vessy(CH)**
Inventor: **Voellmy, Richard**
**7240 SW 124th Street**
**Miami Florida 33156(US)**

(74) Representative: **Dousse, Blasco et al**
**7, route de Drize**
**CH-1227 Carouge/Genève(CH)**

(54) Method for the in-vivo production and testing of proteins by recombinant gene expression in selected host-cells.

(57) Eukaryotic host-cells transformed with stress-inducible gene constructs are inoculated into selected warm-blooded recipient animals multiplied either neat or as tumor fragments. Upon application of stress, either locally (tumor site) or as a whole, under harmless conditions, gene expression occurs and the gene products can be isolated from the body fluids of the animals. The response of the animals to the newly synthesized products can be observed directly, which eliminates all purification problems inherent to first isolating the substance before making physiological tests. Moreover the antibodies raised by the animal against the new product can be isolated by usual means or produced in-vitro from hybridoma strains involving cells taken from immunoorgans of the animals.

FIG. 1

# METHOD FOR THE IN-VIVO PRODUCTION AND TESTING OF PROTEINS BY RECOMBINANT GENE EXPRESSION IN SELECTED HOST-CELLS

## Introduction

The present invention relates to a method for the in-vivo testing of new products of biological interest, e.g. proteins produced by the induced expression of recombinant genes in eukaryotic host-cells. This invention is therefore important for the testing of new medical and pharmaceutical products obtained by industrial genetic engineering methods, e.g. drugs, hormones, enzymes and the like which result from the expression of copies of natural genes or synthetic engineered variants, after incorporation of said genes into recipient cells, cultivation and multiplication of these cells, and ap plication of stress.

It is well known that all new bioactive products of commercial interest must be carefully tested and submitted for approval before being released on the market. Most of these tests are performed in-vitro on cell cultures and in-vivo on laboratory animals. One of the most important criteria in developing and testing new products is purity, i.e. the elimination of undesirable side substances which contaminate the desired product and which may have detrimental or uncontrollable side effects in the tests. Yet, obtaining highly purified products from mixture obtained by methods involving classical genetic engineering techniques, i.e. the broths of cell cultures with their thousands or so different substances, is often difficult, time-consuming and expensive. Another related problem is the obtention of fully competent products. This requirement, for instance with secretable proteins, is sometimes far from evident since, depending on the production process and the selected host-cell system, the proteins obtained may need further post-translational processing (e.g enzymatic cleavage) to become fully competent.

## Summary of the invention

It is now proposed, in order to remedy some of the above deficiencies related to the nature of the selected host-cells, to carry out the inducible expression of genetically engineered genes directly in warm-blooded animals. This procedure is based on inoculating selected warm-blooded animals with cell lines capables of multiplying unrestrictedly therein and harbouring recombinant genes of interest, these genes being expressible within the animal body upon stress. A response therefore occurs in the animal which can be analysed, monitored and the products of expression recovered. This method is summarized in annexed claim 1. It should be noted at this stage that document WO-A-8 700 201 discloses keratinocyte cells modified genetically to form epithelia can be grafted to patients and operate as a source of substances to be directly released to the body. However, obviously, such a local application has very little in common with the present invention in which the injected foreign cells can multiply unrestrictedly in the animal and produce a response much greater than in the reference.

One embodiment of the present invention derives from a technique, disclosed in document PCT EP87 00599, involving transfection of appropriate eukaryotic host-cell lines capable of generating tumors when inoculated to immunodeficient animals with nucleic acid constructions containing at least one gene of interest under the control of a stress inducible promotor. With "normal" cells used as hosts, a transforming gene, e.g. an oncogene, is also added by co-transfection to impart to the cells unrestricted multiplication capacity. Then the transformed cells which are capable of expressing said gene of interest upon induction are inoculated to immunodeficient animals whereby they are transiently replicated in the form of tumors developed by the animals.

Then, according to PCT/EP87/00599, after reaching a given degree of developments, the tumors are removed, the cells are dissociated and placed in culture whereby they can be further multiplied and eventually stressed for generating the desired product. The advantage of this method is speed and efficient cell replication without degeneracy, maintenance of the stress inducible character for a prolonged period, and the direct production of fully competent secretable proteins.

One aspect of the present invention relies on the aforementioned technique. Indeed, it has been now discovered that instead of removing the tumors and thereafter subjecting the dissociated cells to stress, the tumor-carrying animals themselves can be transiently stressed, without visible ill effects, whereby expression of the stress inducible (preferably heat-inducible) foreign genes occurs, and the product is formed in the body of the animals, i.e. it is secreted into the blood stream or the ascite fluid thereof and its physiological effect thereon can be observed directly. In a second embodiment, no visible tumor is grown in response to inoculation. However, after a period of time, a response develops in the animal subjected to stress, as ascertained by the formation in body fluids of substantial amounts of products of expression of said recombinant genes.

It is important to note that the present invention elegantly remedies the problem associated with the need of testing new products in a high purity state. Indeed, in the method of the invention, the desired gene products are the only new ones that should normally form after induction of expression and any off-response of the animal possibly due to some other reason in the growing of the host-cells tumors can be distinguished by using control animals inoculated with identical host-cells not carrying the gene expression construct. Therefore the usual problems of isolating a new product from biofermentors and purifying it free from possible impurities prior to testing are also avoided.

Another advantage of the present method is that the response of the animal to the newly expressed substance may involve the formation therein, of metabolites, e.g. antibodies which may become present in sizable amounts. Therefore, the present invention is also a method for the in-vivo production of antibodies to the newly synthesized products which are made available by extraction from the blood or other fluids of the experimental animals in which they have been raised.

Furthermore, since antibodies are present in the organism of the animal, the cells specifically expressing such antibodies (usually spleen cells) can be identified and isolated. These cells (lymphocyte B) can be used according to usual means to manufacture hybridoma strains, for instance with SP-20 cells, or using other similar methods.

Hybridoma cells can thereafter be used for the production of monoclonal antibodies by standard procedures.

In addition, this invention can serve to provide animal models for the pharmaceutical evaluation of new therapeutic proteins. If a diseased state is present or induced in a test animal and a therapeutic protein to treat this disease is produced in the animal as described above, the IN VIVO activity or potency of the therapeutic protein can be ascertained in terms of its effectiveness in relieving the disease symptoms. In other words, new variant medicament proteins can be tested for modified or increased activity prior to isolation, purification, direct injection and subsequent extraneous utilization.

## Detailed description of the invention

In the present invention, the production of proteins is achieved by inoculating into appropriate animals tumor cells which are able to produce secretable (or trans-membrane) proteins, preferably using a heat-inducible expression system. The general advantages of expressing genes, e.g. human Growth Hormone (hGH) gene, under the control of heat-shock expression elements for the production of gene products have previously been described (Bromley and Voellmy, (1983), Patent Application US serial no 628.588; Bromley et al., (1985), Patent Application EP86/00451; Bromley et al., (1986), Patent Application EP86/8104555; Bromley and Dreano, (1987), Patent Application EP87/81003.3; Dreano et al., (1987), Gene 49, 1-8; Dreano et al., (1987), Virus Res. 8, 43-59). The application of this system to animals inoculated with recombinant cells capable of producing gene products after induction, is of considerable interest. This allows specific protein production at a determined time, and for secretable proteins, potential secretion into the blood stream.

This procedure is thereafter directly applicable to the study of the physiological effects of in-vivo produced gene products, without any prior purification step, thus avoiding potential problems of loss of activity by denaturation, while reducing both cost and time of production.

Moreover, most proteins of therapeutic interest are generally secreted proteins; such proteins frequently require post-translational modifications for optimal activity and immunological specificity. These modifications can be obtained by a specific selection of the recombinant cells, and such matured proteins can be secreted into the blood stream. Moreover, the time to obtain significant quantities of producer cells, as well as the cell culture cost is much reduced using this system.

In the present invention several lines of host cells can be used which can be transformed with DNA vectors carrying the desired stress inducible genes. Most suitable cell lines known by people skilled in the art are convenient. For instance Cl.6 cell lines used in this invention are NIH-3T3 cells co-transfected with plasmid p17hGHdhfr (see Dreano et al. (1986) Gene 49, 1-8) and with the human ras oncogene. These cells can secrete in-vitro 2 -5 $\mu$g of hGH (human growth hormone) per $10^6$ cells over a 20 hrs period that follows a 1 1/2 hr heat shock at 43°C. This is about 1200 times the quantity of hGH correspondingly found in unheated Cl.6 cells.

Another kind of host cells used in this invention are designed Wg6. These are "Wish Cells" (ATCC-CCL 25) transfected with p17ghGHneo. This plasmid contains a genomic BamHI 2.1 kb hGH sequence derived from plasmid pMThGH (see Palmiter et al. (1983) Science 222, 809-814) instead of the hGH cDNA of the plasmid used in the foregoing Cl.6 type cells. Plasmid p17ghGH neo also contains the human hsp 70 promoter for driving the hGH gene and a neomycin resistance gene driven by the SV40 promoter. Wg6 cells are derived from a clone exhibiting resistance to G418 antibiotic (a modified neomycin antibiotic) and secrete in culture media about 3 $\mu$g of hGH/$10^6$ cells after heat shock.

Another line of cells used in this invention are designated EMS8. For this, Cl.6 (see above) were retransfected with plasmid p17MSneo comprising sequences of the Hepatitis B virus coding for the surface antigen protein (HBsAg) under control of the human hsp70 promoter, and the transcription unit for expressing modified neomycin resistance (Dreano et al. (1987) Virus Res. 8, 43-59). EMS8 is a clone resistant to G418; in culture media it secretes, after heat shock, 5 $\mu$g of hGH and 270 ng of HBsSg/$10^6$ cells.

H14.300 is a designation for another cell line used in this invention. The H14.300 cells are Chinese Hamster Ovary (CHO) cells transfected with plasmid p17hGHdhfr (see PCT/EP87/00599) and cultured in media containing increasing concentrations of methotrexate in order to amplify the number of copies of the dihydrofolate reductase (dhfr)gene and adjacent sequences, in this case the hsp70 driven hGH hybrid gene. The cultured cells are capable to secrete up to 45 $\mu$g of hGH/$10^6$ cells after application of stress.

In addition to the study of the physiological activity of gene products, the response to inoculation in animal also involves immunoactivity. For example, it has been observed that after two expression induction steps, three weeks after inoculation of line 6 host-cells, production of anti-hGH antibodies took place in said animals. This obervation therefore allows one to obtain specific antibodies without prior injection of the purified product, and without addition of adjuvant which is normally employed to increase immunological response. In addition, this method has the advantages of presenting secreted (or trans-membrane) proteins in a cellular and more physiological manner to the immune system, leading probably to a more effective and more specific immune response.

More specifically, eucaryotic cell lines capable of generating tumors (either tumor cells or "normal" cells transfected with an oncogene or an oncogenic viral element) are trans fected with nucleic acid constructions containing at least one gene of interest, placed under the control of a stress-inducible promotor, preferably a heat-shock promotor. Then, the said transformed cells are inoculated subcutaneously (s.c.) or intraperitoneally (i.p.) into animals, whereby they replicate to form tumors in the animals. We have already demonstrated in previous work that the rates of cell multiplication were very high without tumor degeneracy, i.e. portion of the tumors can be reinjected and furnish, in succession, a series of transplantable new generations up to about 30 or more. Such tumors can be trypsinized and re-inoculated into Petri dishes, or into biofermentors, without any apparent loss of their capacity to express the gene of interest under stress (Bromley et al., (1986), Patent Application EP86/810455.).

The warm-blood animals which can be inoculated according to the present invention are preferably immunodeficient or immunosuppressed chemically, e.g. with cyclosporine. These animals include mice, nude mice (immunodeficient), Sencar mice (sensitive to carcinogens), balb/c mice, rats and rabbits. However other animals like guinea pigs, hamsters or larger animals like goats, horses, and the like can also be used. Inoculation can be performed sub-cutaneously (sc) or intraperitoneously (ic).Inoculation performed sc leads generally to the formation of tumors which can be isolated, comminuted and the comminution products reused for subsequent inoculations; however sc inoculation can also lead to animal response without tumor formation. Conversely, ip inoculation generally produces response without tumor; but this is not always the rule and sometimes tumors may also form in the animal.

Some specific experiment results are discussed below.

For instance, after previous passage in nude mice, genetically engineered and transformed cells (e.g. line 6 cells co-transfected with plasmids containing, respectively, the human H-ras oncogene, and the hGH gene under the control of the human hsp 70 promotor) were directly injected s.c. into Sencar strain of mice (Swiss derived strain, see Experimental section), which is not genetically immunodeficient; Sencar mice also developed s.c. tumors in around 80% of all performed injections.

S.c. tumors were also obtained in rats which received cyclosporine as a chemical immunosuppressant, and were s.c. transplanted with pieces of about 75 mm$^3$ of nude mouse tumor. Non-cyclosporine treated rats regressed the transplant quite rapidly, in around 2 weeks, while rats which received one injection of cyclosporine took an average of 4 weeks to regress their tumors.

The experiments which support the claimed method include the following embodiments; animals carrying tumors were incubated at 43°C for 10 to 60 min, and blood samples were taken after different periods of post- induction from behind the eyeball; levels of secretion of hGH in the serum of animals was subsequently measured. Maximum secretion is reached 5 h after induction, and heat induction can be repeated a number of times on a daily basis in mice. Secretion levels in Sencar mice and in rats are quite variable, with levels in the rat being relatively low.

In addition, after repeated induction treatments, antibodies against gene products, e.g. hGH were detected in the blood of both Sencar mice and rats carrying tumors.

In addition to the above, the following results were noted:

Inoculation of Cl.6 into Sencar mice, balb/c mice, rabbits and rats lead to ultimate anti-hGH production. With Sencar mice inoculation was done either sc or ip and the antibody was present in the blood and in ascite fluids.

Using Wg6 cell line in rabbits, the formation of antibody occured after inoculating cells cultivated in-vitro. Since Wg6 cells harbour a plasmid containing a "natural" genomic hGH gene, it is of interest to note that the production of hGH occurs as well as in the case of Cl.6 in which the gene is cDNA. Furthermore, antibody production is effective in rabbits even with human recombinant producer cells.

The inoculation of EMS8 cells into Sencar mice demonstrated the operability of a system comprising two independent genomic transformations, i.e. the expression of hGH with corresponding antibody formation and, simultaneously, the expression of HBsAg gene with corresponding anti-HBsAg formation.

Details of the various embodiments of this invention are found hereafter; these details are presented with reference to the annexed figures.

## Brief description of the figures

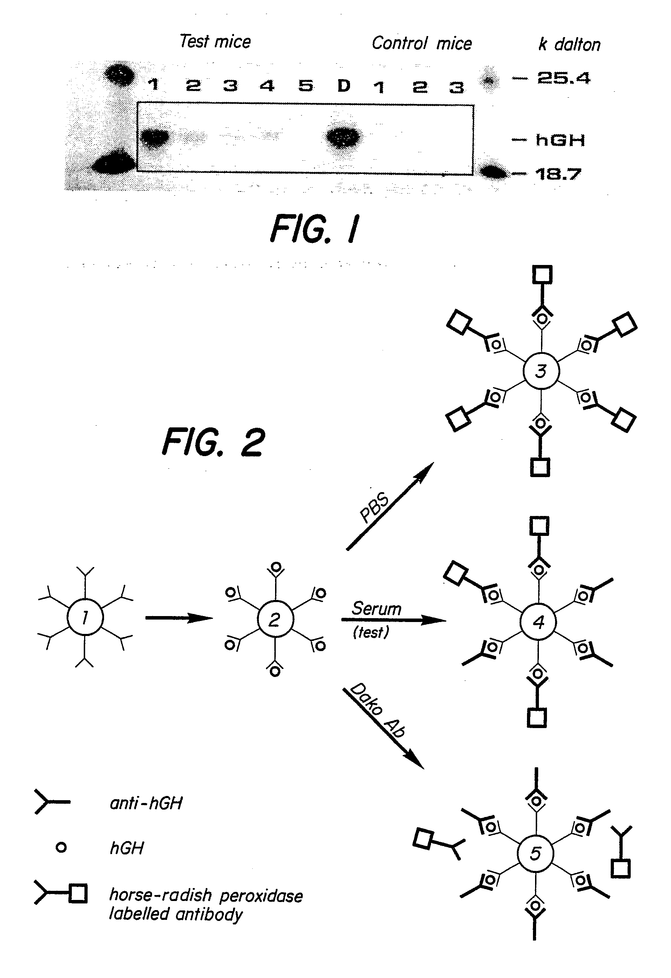

Fig. 1 is an autoradiography of a 15%-acrylamide gel electropherogram showing some protein fractions produced after heat-shock by Cl.6 cells cultured in-vitro in the presence of $^{35}$S-methionine. These proteins containing radio-labelled hGH were immunoprecipitated with (i) sera from heat treated (Test mice) Sencar mice carrying line 6 tumors (lines 1 to 5), (ii) rabbit anti-hGH control serum from Dako (D), and (iii) control sera from three normal Sencar mice (control mice 1 to 3).

Fig. 2 is a schematic representation of competition experiments aimed at detecting anti-hGH antibodies in sera from heat treated tumor-carrying animals, using the Sensibead ELISA kit. Beads carrying an immobilizing reagent (1) were reacted with hGH to provide the hGH coated beads (2). Beads (2) were used for competitively analyzing (4) sera from heat treated animals (reagent used was horse radish peroxidase labelled anti-hGH antibodies). (3) constitutes a control experiment with zero percent animal-raised antibody. (5) constitutes another control with 100% antibody (reagent from Dako).

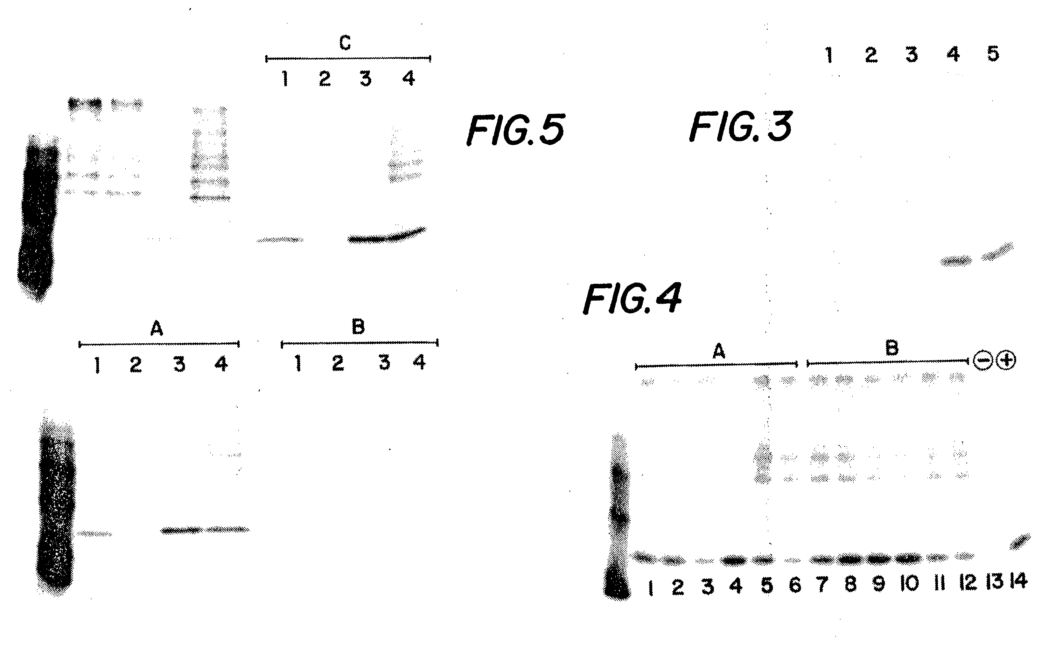

Fig. 3 is an autoradiography of an electropherogram made on 15% acrylamide gel somewhat similar to that of fig. 1, i.e. it shows protein fractions expressed in-vitro by cultured Cl.6 after heat- shock immunoprecipitated by the antibodies developed in body fluids of heat shocked Sencar mice carrying tumors consecutive to ip inoculation with Cl.6. Lane 4 refers to precipitation by blood serum: lane 5 refers to precipitation ascite fluid. Lanes 1-3 refer to sera of mice inoculated identically without developing tumors.

Fig. 4 is similar to the previous figure but refers to immunoprecipitation of protein fractions including hGH by sera of balb/c mice carrying tumors due to Cl.6 sc inoculation. Lanes 1-3 and 7-9 are controls; lanes 4-6 (group A) and 10-12 (group B) refer to heat treated animals. Group B corresponds to results from the same animals as group A but sampled two weeks later. Note that results 11 and 12 are stronger than corresponding 5 and 6 which show that response in the blood of the animals increased in the two weeks following the first sampling.

Fig. 5 is also an autoradiography similar to the previous ones in which protein fractions issued from heat shocked cultures of transformed cells are immunoprecipitated by sera from heat treated animals previously inoculated with said transformed cells. The cell lanes refer to: 1 Cl.6 after heat shock; 2 Cl.6 control (no heat shock); 3 H14.300 after heat shock; 4 Wg6 after heat shock. H14.300 are CHO cells transfected with p17hGHdhfr. A, B and C refer to, respectively, Sencar mice, rats carrying Cl.6 sc tumors and commercial rabbit sera.

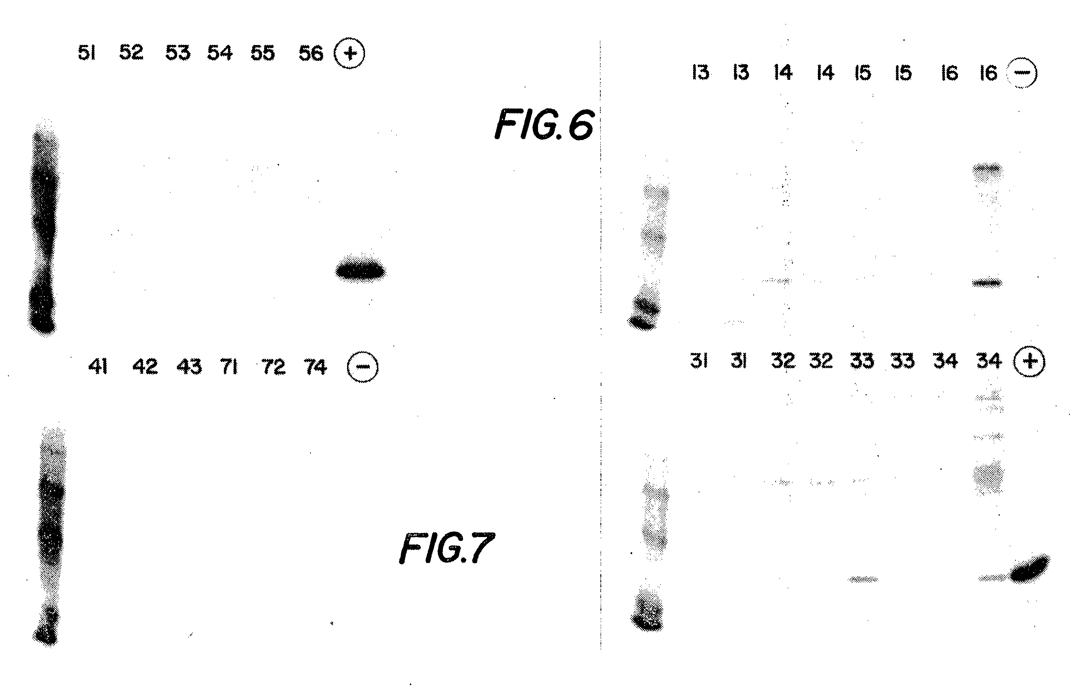

Fig. 6 is also an autoradiography of the results of an electrophoretic separation on 15% acylamide gel of the protein fractions produced after heat shock by H14.300 cells cultured in-vitro in the presence of $^{35}$S-methionine, these proteins being immunoprecipitated by the sera of rabbits inoculated with Cl.6 tumor cells and, subsequently, subjected to heat treatments. The reference numbers 13 to 16 and 31 to 34 correspond to the numbers given to the animals under test (see experimental part). Each pair of numbers corresponds to two consecutive samples from the same animal, the samples being taken at two weeks interval. - and + are for calibration purposes, i.e. sera from non-inoculated mouse and commercial serum, respectively.

Fig. 7 also represents an electropherogram on 15% acrylamide gel of protein fractions produced by H14.300 cells after heat shock, the culture medium containing $^{35}$S-methionine, after precipitation by sera of rabbits inoculated with Wg6 cells raised in-vitro. Lanes 71 and 72 correspond to sera from rabbits injected ip with sodium thioglycolate (TGC) to induce aseptic peritonitis prior to Wg6 inoculation. Lane 74 corresponds to serum of a non TGC-treated rabbit.

**Experimental (Examples)**

**Construction of p17ghGHneo**

5 $\mu$g of pMThGH (Palmiter et al., 1983, Science 222, 809-814) and 5 $\mu$g of p17hGHneo (Dreano et al., 1988, Bio/Technology 6, 953-958) were digested with BamHI. The restriction fragments were separated by electrophoresis on 1% agarose gel. A 2.1 kb hGH DNA from pMThGH containing the genomic hGH gene including four introns was selected as well as a 6.0 kb fragment of p17hGHneo containing: i) the hsp70promotor, ii) a pBR322 derived bacterial vector carrying the origin of replication and the ampicillin resistance gene, and iii) the transcription unit of the neomycin resistance gene under the control of SV40 transcriptionnal signals (Southern and Berg, 1982, In Gluzman, Y. (Ed.) Eucaryotic Viral Vector, Cold Spring Harbour Laboratory Press, New York). These fragments were purified using Geneclean procedure (Geneclean ® kit, Bio 101, La Jolla, Ca). The purified fragments were ligated with T4 DNA ligase and used to transform Escherischia Coli, HB101 strain as described in Maniatis et al., 1982, Molecular Cloning, Cold Spring Harbor Laboratory Press, New York. After selection on L broth medium containing ampicillin, DNA of E. Coli neomycin resistant colonies were analysed by restriction mapping. A plasmid containing the genomic gene of hGH in the right orientation under the control of the hsp70 promotor and the neomycin resistance gene was characterized and designated p17ghGHneo.

**Selection of Wg6 cells**

A quantity of about $10^6$ Wish cells (ATCC-CCL 25) was subjected to transfection using the CaCl$_2$ procedure of Graham and van der Eb, 1973, Virology 52, 456-467, with 10 $\mu$g of p17ghGHneo. Two days later, the cells were split, diluted 1/10 to 1/20 and cultured in Dulbecco's modified Eagles' medium supplemented with 10% fetal calf serum and containing 1.500 $\mu$g G418 (Gibco) per ml. Three weeks later, visible clones had formed; they were expanded and heat treated. Supernatants were tested for hGH as disclosed below under section 2. One cell line, designated Wg6, was selected. Wg6 can secrete in the culture medium around 3 $\mu$g of hGH per million cells after heat shock.

**Selection of EMS8 cell line**

A quantity of about one million Cl. 6 cells (Dreano et al., 1986, Gene 49, 1-8) was transfected using the foregoing Graham and van der Eb procedure with 10 $\mu$g of p17MSneo (Dreano et al., 1987, Virus Res. 8, 43-59). This plasmid contains the hepatitis B virus surface antigen (HBsAg) under the control of the human hsp70 promotor and the neomycin resistance gene driven by SV40 transcription signals (Southern and Berg, 1982, In Gluzman, Y. (Ed.) Eucaryotic Viral Vector, Cold Spring Harbor Laboratory Press, New York). Two days later, cells were split, diluted 1/10 to 1/20 and cultured in Dulbecco's modified Eagles' medium containing 400 $\mu$g G418 (Gibco) per ml. Three weeks later, visible clones had formed and were expanded in tissue culture. The multiplied cells were heat treated, and supernatants were tested for hGH and HBsAg concentrations as indicated before for hGH but using an ELISA kit for HBsAg (EIA-kit from Abbott). A cell line, designated EMS8, was selected. EMS8 cells can secrete in the culture medium around 3 $\mu$g of hGH and 270 ng HBsAg per million cells after heat shock.

**Selection and amplification of CH0 cell line transfected with p17hGHdhfr (H14.300 cell line).**

About a million dihydrofolate reductase (dhfr)-deficient CHO cells (Urlaub and Chasin, 1980, Proc. Natl. Acad. Sci. USA 77, 4216-4220) were transfected using the aforementioned CaCl$_2$ procedure with 10 $\mu$g of

p17hGHdhfr (Dreano et al., 1986, Gene 49, 1-8). This plasmid contains the hGH gene under the control of the human hsp70 promotor and the dhfr gene driven by SV40 transcription signals (Subrami et al., 1981, Mol. Cell. Biol. 1, 854-864). Growth was carried out in non-selective condi tions, i.e. in Ham's F12 medium supplemented with 10% of fetal calf serum and 70 $\mu$g of proline (Gibco) per ml. Two days later, the cells were split, diluted to 1/10 or 1/20 and cultured in Dulbecco's modified Eagles' medium containing 70 $\mu$g of proline per ml. Three weeks later, visible clones had formed which were expanded in tissue culture. The cell lines were heat treated, and supernatants were tested for hGH as above. A cell line, named H14 was found to be able to secrete in the culture medium around 1 $\mu$g of hGH per million cells. H14 cells were cultured in media containing increasing concentrations of methotrexate, i.e. with 0.5 $\mu$M at start and 30 $\mu$M at end (4 fold increase). Since methotrexate is an antagonist to dhfr operation, the selection pressure resulted into amplification of the transfected dhfr gene and associated DNA (Alt et al., 1978, J. Biol. Chem. **253**, 1357-1370; Kaufman et al., 1985, Mol. Cell. Biol. 5, 1750-1759). The amplified cell line was designed H14.300. It is capable, after heat induction, to secrete 45 $\mu$g hGH per million cells.

## 1. Transplantation of tumors into animals

· We report below the results of transplantation procedures of Cl. 6 cells into nude, balb/c and Sencar mice, and into immunosuppressed rats. Other genes of interest coding for a variety of bioactive products, e.g. enzymes, hormones, inhibitors, antigens, antibodies, etc..., can also be placed under the control of hsp 70 promotor sequences, in similar DNA constructions as described hereafter. In addition, other immunosuppressed (or not) animals can be used, such as guinea pigs, hamsters, rabbits or perhaps even goats, horses, etc.

Transplantation into animals with the Cl. 6 cell line has been already described in Patent Application EP86/00451. Plasmid constructions, cells selections and characterizations are disclosed in Dreano et al., (1986), Gene 49, 1-8 and in the above Patent documents. Nude (nu/nu) mice aged 4 to 6 weeks, from IFFa-Credo (L'Arbresle, France) were housed in macrolon cages with a cover filter, and placed in an air-filtered cabinet. They were fed an autoclaved diet and normal tap water. Portions of $10^6$ genetically modified cells were trypsinized, rinsed twice with Dulbecco's modified Eagle's medium without serum, and resuspended in about 0.1 ml of the same medium; these suspensions were used for subcutaneous injections. Briefly, $10^6$ cells, in 0.1 ml of culture medium without serum, were injected s.c. in the dorsal area of nude mice. The first tumor was clearly visible 2 weeks after inoculation. When a tumor reached a size of about 20 mm or more (about 2 weeks after injection), tumors were dissected, and minced finely in culture medium, and portions were reinjected s.c. into other nude, balb/c or Sencar mice to provide successive generations of tumors. In other experiments, similar portions were injected ip into nude and Sencar mice. The reason for producing a large number of successive generations in two-fold: 1. To check the overall stability of the cellular progeny; 2. it is important to permanently maintain the cell cultures to ensure constant production of the proteins of interest. Tumors were always obtained in nude mice, and in almost all attempts with Sencar mice. Battelle-Geneva breeds the Sencar strain of mice, an albino mouse derived from the Swiss mouse by selection for its sensitivity to skin carcino gens (the term Sencar comes from "Sensitive to Carcinogens"). Contrary to the nude mice, this strain of mouse is not genetically immunodeficient. To obtain tumors in rats, animals were immunosuppressed by five daily consecutive injections of cyclosporine (60 mg/kg, Sandoz) and pieces of about 75 mm³ of tumor from nude mice were introduced under the skin. After that, animals were no longer treated with cyclosporine. After two weeks, the tumor had regressed in non-cyclosporine treated rats, while tumors developed in cyclosporine-immunosuppressed rats.

## 2. Heat treatment of the animals and measurement of hGH protein and anti-hGH in body fluids

Animals carrying tumors were heat treated at 43° C in a ventilated incubator for 10 to 60 min. The first induction experiments were performed a few days after injections. Under these conditions the internal temperature of nude mice increases gradually up to 41° C. Post-incubation resting periods were performed under normal conditions (room temperature between 19 and 24° C). Blood samples were taken from the vein behind the eyeball of animals and were put into eppendorf tubes, with or without heparin. After coagulation (in the absence of heparin) blood samples were spun at 3,000 rpm for 3 min, and the supernatants were assayed for the presence of hGH using an ELISA kit (sensibeads, Terumo Medical Corporation, Japan). The technique, which is detailed in the instructions provided with the kit, is based on the same kind of reactions disclosed below in connection with anti-hGH determination, the only difference being that with anti-hGH the response generated by the marker is in direct proportion to the amount of hGH, whereas with anti-hGH, the response is inversely proportional to the amount of anti-hGH. Blood samples with heparin were spun at 3,000 rpm for 3 min, and the supernatants were once again spun at 10,000 rpm for 1 min, and the sera were assayed for anti-hGH antibodies using the same ELISA kit, but employing the following procedure (see fig. 2). Beads (1) coated with an immobilized mouse anti-hGH monoclonal antibody were incubated for 2 hours at room temperature with 0.3 ml of phosphate buffer saline (PBS) solution containing 30 ng hGH/ml. As control, one bead was incubated with PBS alone without hGH. After washing with PBS containing 0.05% Tween 20, beads were incubated overnight at 4°C with known dilutions of rabbit anti-hGH serum (polyclonal, from Dako) or dilutions of sera from normal, or heat treated tumor-carrying animals (4) (dilutions of sera were made in PBS containing 1% of normal mouse serum). To establish a calibration graph, the same reactions were performed, using in place of the animal sera, known standard dilutions of rabbit anti-hGH (DAKO). Then, the beads were washed and competitively incubated 2 hours at room temperature with horse-radish peroxidase-conjugated mouse anti-hGH antibodies (monoclonal), and the detection was obtained after reaction of the orthophenylene diamine reagent, and measurement of the optical density at 490 nm.

**2.1 Amounts of hGH in the blood of nude and Sencar mice carrying Cl. 6 Tumors resulting from sc inoculation**

Mice carrying Cl. 6 tumors (from generation 20 to 25 tumor passages in nude mice) were heat treated for 10 to 60 min at 43°C. Blood samples were taken at from 0 to 29 h after induction, and were assayed for hGH concentration as disclosed under 2. Table I shows that hGH was found in the blood stream of nude mice. A heat treatment of 20 to 30 min appears optimal, 60 min is too long, and 10 min is insufficient. Maximum secretion is reached in about 5 hrs after induction, and after 29 hrs hGH appears to have been metabolised.

TABLE I

| HS Treatment for | Post incub. time | ng hGH/ml of blood | | | | | |
|---|---|---|---|---|---|---|---|
| | | Mouse No | | | | | |
| (min) | (hrs) | 1 | 2 | 3 | 4 | 5 | 6 |
| 0′ | | none | none | | | | |
| 10′ | 5 | none | none | | | | |
| 10′ | 8 | none | 5 | | | | |
| 20′ | 5 | 135 | 140 | | | | |
| 20′ | 8 | 125 | 130 | | | | |
| 30′ | 2 | 90 | 38 | | | | |
| 30′ | 5 | 130 | 120 | 125 | 58 | 104 | 112 |
| 30′ | 8 | 80 | 90 | | | | |
| 30′ | 29 | 3 | 6 | | | | |
| 60′ | 5 | 116 | 80 | | | | |
| 60′ | 29 | 4 | 3 | | | | |

Similar experiments are illustrated in Table II. Nude and Sencar mice carrying Cl. 6 tumors (respectively 25th and 15th generation) were heat treated daily, five consecutive days, for 30 min at 43°C at 24 h interval. In nude mice hGH was found after each induction at a high level of secretion. hGH is also secreted, but less, in Sencar mouse no. 1. However there was practically none in mouse no 2; this animal had perhaps developed antibodies against hGH; the stress promotor could have been somewhat leaky (induced) due to the normal immuno-response of the immunological system, and the hGH possibly produced would then be blocked via induced antibodies.

TABLE II

| | ng hGH/ml of blood | | | |
|---|---|---|---|---|
| days of treatment | nude mouse | | Sencar mouse | |
| | 1 | 2 | 1 | 2 |
| 1st | 110 | 30 | 45 | 10 |
| 2nd | 150 | 70 | 17 | 5 |
| 3rd | 220 | 115 | 55 | <5 |
| 4th | 115 | 115 | 50 | -- |
| 5th | 75 | 190 | 30 | <5 |

## 2.2 Intraperitoneal injection of tumor fragments in nude mice

Nude mice were injected ip with Cl.6 tumors fragments, whereby intraperitoneal tumors developed. They were heat treated nine days after injection, for 20 min at 43° C and, 5 hours later, blood samples were taken and assayed for hGH concentration as above. Table III shows that significant levels of hGH, comparable to those obtained with sc tumor, were found in the blood stream of such animals. It should be noted that hGH production occurs only after heat induction.

TABLE III

| HS treatment for | Post incub. time | ng hGH / ml of serum | | |
|---|---|---|---|---|
| (min) | (hrs) | Mouse No | | |
| | | 1 | 2 | 3 |
| none | 5 | none | none | |
| 20′ | 5 | .150 | 138 | 131 |

## 2.3. Anti-hGH antibodies in the blood stream of Sencar mice

Tumor Cl. 6 cells from nude mice tumors (29th generation) were injected subcutaneously in the back area of 5 Sencar mice. Animals were heat treated for 30 min twice a week during one month. Three animals (T1, T2 and T3) developed large tumors, while two others (T5 and T6) had tumors which regressed after two weeks. The mice were sacrificed and blood was collected. Serum fractions were prepared and tested for the presence of anti-hGH antibodies, using the competition procedure described above under section 2. Sencar mice carrying large tumors produced significant amounts of anti-hGH antibodies that compete with the monoclonal anti-hGH peroxidase-conjugated antibodies from the ELISA kit, while the competition with the sera of the mice which have regressed the tumor were weaker (Table IV). In Table IV are also presented the results for three control mice (C1, C2 and C3) which did not carrying tumors. The results from three dilutions of Dako antibody are also presented.

TABLE IV

| Test Solutions | Mice | Dilutions | OD 492 nm |
|---|---|---|---|
| PBS | | --- | 1.066 |
| Dako(without hGH) | | 1/5 | 0.062 |
| Dako Ab | | 1/500 | 0.695 |
| Dako Ab | | 1/50 | 0.279 |
| Dako Ab | | 1/5 | 0.167 |
| Serum | C1 | 1/5 | 0.995 |
| Serum | C2 | 1/5 | 1.009 |
| Serum | C3 | 1/5 | 1.344 |
| Serum | T1 | 1/5 | 0.278 |
| Serum | T2 | 1/5 | 0.508 |
| Serum | T3 | 1/5 | 0.787 |
| Serum | T4* | 1/5 | 0.841 |
| Serum | T5* | 1/5 | 0.833 |

* Tumors in mice 4 and 5 had regressed two weeks after inoculation. T for Test mice and C for Control mice.

The antibodies produced in mice 1-5 were also identified by immuno precipitation with isotope-labelled hGH. This is illustrated in fig. 1. Cl. 6 parental cells (i.e. cells not injected into animals) were cultured in media containing $^{35}$S-methionine. The cultures were heat-shocked and radio-labelled hGH was isolated therefrom by usual means.

More specifically, labelled hGH was produced by incubation of heat-treated $10^6$ Cl.6 cells in growth medium containing 40 $\mu$Ci/ml of $^{35}$S-methionine, for a 15 h period at 37° C. Sera from normal or tumor-carrying animals were obtained as previously described. Immunoprecipitation was carried out as described in Dreano et al., (1986), Gene 49, 1-8. After boiling, immunoprecipitated fractions were analyzed on SDS - 15% - acrylamide gels.

Fig. 1 shows that the hGH produced by the parental cells was immunoprecipitated by antibodies produced in large tumor-carrying Sencar mice (T1 to T3) as well as, under the same conditions, with the polyclonal rabbit anti-hGH serum (D = Dako anti-hGH antibodies). It is interesting to note that the serum of one of the two mice (T4) in which the tumor had regressed, contains enough anti-hGH antibodies to immunoprecipitate the hormone. Thus, short periods of immunization employing this system appear sufficient to produce antibodies. Amounts of $^{35}$S-hGH immunoprecipitated by mouse 1 serum were not very different from the amounts precipitated with control rabbit serum and, taking into account that the protein-A sepharose is more specific for the attachment of rabbit antibodies than for mouse antibodies, antibody production using this system would appear to be very efficient. This process can thus be used as a general method for antibody production without prior purification of the antigen. The very high degree of specificity of the induced antibodies is demonstrated by the extreme purity of immunoprecipitated hGH (narrow bands).

**2.4. Anti-hGH antibodies in the ascite fluid of Sencar mice**

Some of Sencar mice inoculated ip with Cl.6 tumor developed large abdomen tumors within an average of 10 days while others did not. The reasons for the differences were not studied. The mice were sacrified and samples of blood and ascite fluid were taken out and tested for anti-hGH antibody concentration by immunoprecipitation using labelled hGH produced by Cl.6 cells (produced as described above).

Immunoprecipitated fractions electrophoresed on SDS-acrylamide gels are shown in Fig. 3. Thus, large quantities of anti-hGH antibodies are present in the sera of animals inoculated ip with Cl.6 tumors (lines 1 to 4). In addi tion, antibodies are detected in the ascite fluid of mouse with ip tumor (line 5). Similar results were observed in ascite fluids and blood sera from Sencar mice inoculated ip with EMS8 cells. Since the volume (up to 8 ml) of ascite fluid is much greater than that of the blood of the animal, using the ic inoculation route is advantageous for the production of large quantity of specific antibodies.

## 2.5 Anti-hGH antibodies in blood stream of Balb/c mice

Six Balb/c mice were injected at time t0 (zero) with Cl.6 tumor (see section 1 above) and, after two days, 3 of them were heat treated twice a week at 43°C for 20 min for two weeks. The other 3 were left untreated. Animals regressed their tumors in around two weeks. Then, the six mice were re-injected three and six weeks after t0 with Cl.6 tumors and the three test animals were heat treated in a similar manner, while the three controls were left alone.

Three months later, blood samples were taken and sera were analyzed by immunoprecipitation (as above). Results are shown in Fig. 4. Group A and B of results refer to two series of blood sampling at two weeks interval. Lanes 1-3 and 7-9 concern control mice; lanes 4-6 and 10-12 concern heat shocked test animals. Lanes 13 and 14 are -anti-hGH (non-inoculated mouse) and +anti-hGH (Dako serum raised in rabbits) calibrations, respectively. These results demonstrate that heat treated and control mice both produced anti-hGH antibodies, the amount thereof produced by the test animals being only slightly greater than that from the controls. This indicates that stressing of the injected foreign cells occured during rejection of the tumors by the non-immunosuppressed animals without heat application, thus activating the heat shock system and leading to the secretion of hGH in the blood stream and in anti-hGH antibody production, this being so even in non-heat treated mice, especially when blood samples are taken a long time after the initial tumor injection.

## 3. Expression in-vivo after heat treatment of a rat carrying a line 6 tumor

Pieces of about 75 mm³ of Cl. 6 tumor, from nude mice tumors (33rd generation), were transplanted subcutaneously into Sprague Dawley albino rats (Iffa-Credo, France). The transplantation was performed in 4 rats; 1 normal rat, and 3 rats chemically immunosuppressed, using five daily s.c. injections of 60 mg/kg of cyclosporine (Sandoz, Bennet et al., Cancer Res. 45, 4963-4969). Tumor fragments were inserted under the skin of the rats. After tumor formation (around 7 days), animals were heat treated for $\frac{1}{2}$ to 1 hr and sera were tested for hGH production IN VIVO. Table V shows that hGH was found in the blood of animals, especially after an induction period of 30 min at 43°C and, as seen for nude mice, with a maximum after 5 h of resting at 37°c after heat-shock (HS).

TABLE V

| | hGH ng/ml of sera of rats | | | |
|---|---|---|---|---|
| | 1 | 2 | 3 | 4 |
| | | + cyclosporine | | no-cyclosporine |
| HS period (min) | 30 | 45 | 60 | 30 |
| Time after HS | | | | |
| 0 h | -- | -- | -- | 4 |
| 2 h | -- | 5 | -- | -- |
| 5 h | 10 | 6 | -- | -- |
| 8 h | 2 | 3 | 2 | -- |
| 20 h | -- | -- | -- | 3 |

Amounts of hGH found in rats are lower than those found in nude mice, but it should be noted that rats were not cyclosporine treated after tumor injection, and tumors reached a maximum of 5 cm diameter in 200 to 250 g animals, instead of up to 3 cm diameter tumors in approximately 20 g nude mice.

## 3.1 Anti-hGH antibodies in the sera of rats carrying tumors

Characterization of the products identified in the body fluids of rats was effected as described before in the case of mice, i.e. using immunoprecipitation with labeled hGH obtained by stressing strains of transfected cell lines cultured in the presence of radioactive methionine. The cell lines used were the aforementioned Cl.6, Wg6 and H14.300. Fig. 5 illustrates the comparative production of hGH by the three kinds of cells by immunoprecipitation with commercial anti-hGH raised in rabbits. Other immunoprecipitants whose effect is illustrated here are sera from Sencar mice and rats carrying Cl.6 tumors.

The electropherograms of Fig. 5 indicate that the concentrations of anti-hGH in the Sencar and rabbit sera are high; hence, immunization against recombinant cells antigens is very efficient. In contrast, with rats the concentration of antibody in serum is quite lower. Sera from the above rats (rats 3 and 4 in Table V, labelled R3 and R4) were tested for anti-hGH antibodies as described above. Table VI shows that no anti-hGH antibodies were detected two weeks after tumor injection (5 h after the second induction). The presence of antibodies was however detected three to four weeks after injection, as fixation of the anti-hGH conjugated to peroxidase was inhibited by sera taken at this time. It is interesting to note that the non-cyclosporine treated rat, in which the tumor had regressed in less than two weeks, also produced anti-hGH antibodies.

Table VII shows the results of a similar experiment indicating that: (i) normal rat sera contain products which interact non-specifically with hGH linked to the anti-hGH antibodies (NR and R4, R5), (ii) rats which did not receive cyclosporine produce competitive antibodies (R7) or not, (R6) and Cl. 6 injected rats treated with cyclosporine synthesize different levels of anti-hGH antibodies (R2>R3>R1), and production starts from the third week after cell inoculation.

TABLE VI

| ng hGH | test (control AB of serum of rat) | dilutions | OD 490 nm |
|--------|-----------------------------------|-----------|-----------|
| 10 | PBS (Blank) | -- | 0.540 |
| 0 | Dako Ab | 1/20 | 0.008 |
| 10 | Dako Ab | 1/500 | 0.402 |
| 10 | Dako Ab | 1/100 | 0.209 |
| 10 | Dako Ab | 1/20 | 0.129 |
| 10 | R3 2 wks | 1/5 | 0.535 |
| 10 | R4 2 wks | 1/5 | 0.577 |
| 10 | R3 3 wks | 1/5 | 0.355 |
| 10 | R4 3 wks | 1/5 | 0.310 |
| 10 | R3 4 wks | 1/5 | 0.344 |
| 10 | R4 4 wks | 1/5 | 0.290 |

TABLE VII

| ng hGH/bead | test solutions | cyclosporine treatment | line 6 injection | dilutions | OD 492 nm | |
|---|---|---|---|---|---|---|
| | | | | | 3rd week | 4th week |
| 10 | PBS | | | -- | 1.029 | |
| 0 | Dako | | | 1/100 | 0.000 | |
| 10 | Dako | | | 1/5000 | 0.819 | |
| 10 | Dako | | | 1/1000 | 0.855 | |
| 10 | Dako | | | 1/500 | 0.503 | |
| 10 | Dako | | | 1/100 | 0.266 | |
| 10 | R1 | + | + | 1/5 | 0.401 | 0.368 |
| 10 | R2 | + | + | 1/5 | 0.218 | 0.164 |
| 10 | R3 | + | + | 1/5 | 0.426 | 0.291 |
| 10 | R4 | + | - | 1/5 | 0.350 | 0.408 |
| 10 | R5 | + | - | 1/5 | 0.403 | 0.414 |
| 10 | R6 | - | + | 1/5 | 0.492 | 0.436 |
| 10 | R7 | - | + | 1/5 | 0.424 | 0.317 |
| 10 | NR | | | 1/5 | 0.523 | |

These results demonstrate clearly that mice and rats were able to raise antibodies against a protein produced, on induction, by cells multiplying inside the animal, and that this can be a general method to produce polyclonal antibodies against such proteins when they are recognized as immunogens.

## 4. Anti-hGH antibodies in the blood stream of Rabbits inoculated with tumor fragments

In a first experiment, four rabbits (numbered 13-16) were treated five consecutive days with cyclosporine and in jected sc with Cl.6 tumor fragments (from nude mice). One week following the injection, they were heat treated twice 30 min at 43°C. The tumors which formed disappeared in a few weeks and the animals were re-injected about once a month for six consecutive times. The sixth injection involved a relatively large quantity of tumor fragments (1 full tumor per rabbit). The resulting sc tumor remained visible for up to 8 months. Then, the inoculated animals were subjected to heat treatment at various moments after injection, i.e. from about 2 weeks to 3 months of time. Samples of sera were taken 10 months after the first tumor injection and analyzed by immunoprecipitation.

In a second experiment, rabbits (numbered 31-34) were given only one sc large injection with Cl.6 tumor fragments (from nude mice), and were also heat treated at different intervals of time varying from 2 weeks to 3 months. Sera were analysed 4 months after the initial injection by immunoprecipitation using labelled hGH produced by H14.300 cells (Fig.6). Similar analysis performed with labelled hGH produced by Cl.6 cells gave inconsistent results because a great number of proteins present in the supernatants of the cells did precipitate. The results of Fig. 6 show that anti-hGH antibodies were detected in the sera of the eight rabbits under test. Levels of production are however rather low and depend on parameters such as quantity of injected tumor cells, number of tumor injections, number and time of heat treatment, etc...

## 5. Anti-hGH antibodies in blood stream of Rabbits injected with human tumor cells

Two rabbits were injected with 5 ml of a 5% solution of thioglycollate (TGC) to induce an aseptic peritonitis. Four days later, the 2 rabbits and a non-TGC treated rabbit were inoculated ip with about $30.10^6$ Wg6 cells. The 3 rabbits were heat treated 2 and 5 days later, and blood samples were taken after 3 more weeks. Sera were analyzed by ummunoprecipitation using labelled hGH from H14.300 cells. Fig. 7 shows that weak bands were present at the Mw of the hGH complex. This route of injection of recombinant cell lines therefore activates the immune response system and leads to the synthesis of specific antibodies directed against a heat induced secretable hybrid protein.

## 6. Anti-HBsAg antibodies in blood stream of Sencar mice

Cl.6 cells were transfected again with p17MSneo containing the coding sequence for the hepatitis B virus surface antigen (HBsAg) driven by the human hsp70 promotor and the neomycin resistance gene under the control of SV40 promotor. A neomycin selected clone, named EMS8, was found to secrete around 5 μg and 270 ng per million cells of respectively hGH and HBsAg.

This cell line was inoculated into nude mice, and tumor fragments were injected sc into Sencar mice. Some animals were heat treated and were re-injected once five weeks later. Controls were not heat treated.

Anti-HBsAg antibody production was analyzed two months after the first tumor injection employing a competition procedure similar to those developed for anti-hGH antibodies. Briefly, beads coated with an immobilized mouse anti-HBsAg monoclonal antibody were incubated for 5 hours at 37°C with 0.25 ml of culture medium containing 2.5% fetal calf serum and 10 ng per ml of HBsAg. As a control, one bead was incubated with complete medium without HBsAg. After washing with PBSIX, 0.1% BSA, 0.05% Tween 20, beads were incubated overnight at room temperature with either known dilutions of rabbit anti-HBsAg serum (polyclonal from Behring) or dilution of sera from normal mouse sera, control sera or sera from heat treated tumor carrying animals. After washing, the beads were competitively incubated for 5 hours at 37°C with horse-radish-peroxidase-conjugated mouse monoclonal anti-HBsAg anti body, and measurements of optical density at 490 nm were made after reaction with orthophenylene diamine.

In this experiment, results summurized in Table VIII show that at least 2 mice (Heat shock Sencar 1 et 3) produced significant levels of anti-HBsAg antibodies which did compete with the monoclonal anti-HBsAg antibody, indicating that HBsAg secreted by recombinant cells is recognized as an antigen. In addition, anti-hGH antibodies were found in the sera of each mice tested, already after the first EMS8 tumor injection. Under the conditions experimented, the immunoresponse to hGH is stronger than that to HBsAg. Indeed HBsAg is a very large complex macromolecule which may have difficulties migrating to the blood vessels.

TABLE VIII

| ng HBsAg/ml | test (control AB of serum of Sencar mice) | dilutions | OD 490 nm |
|---|---|---|---|
| 10 | PBS | --- | 0.632 |
| 0 | Behring Ab | 1/5 | 0 |
| 10 | Behring Ab | 1/5 | 0.032 |
| 10 | Behring Ab | 1/50 | 0.026 |
| 10 | Behring Ab | 1/500 | 0.200 |
| 10 | Behring Ab | 1/5000 | 0.489 |
| 10 | Normal Sencar | 1/5 | 0.563 |
| 10 | Control Sencar 1 | 1/5 | 0.707 |
| 10 | Control Sencar 2 | 1/5 | 0.677 |
| 10 | Control Sencar 3 | 1/5 | 0.624 |
| 10 | Control Sencar 4 | 1/5 | 0.621 |
| 10 | Control Sencar 5 | 1/5 | 0.604 |
| 10 | Heat Shock Sencar 1 | 1/5 | 0.296 |
| 10 | Heat Shock Sencar 2 | 1/5 | 0.698 |
| 10 | Heat Shock Sencar 3 | 1/5 | 0.192 |
| 10 | Heat Shock Sencar 4 | 1/5 | 0.631 |
| 10 | Heat Shock Sencar 5 | 1/5 | 0.619 |
| 10 | Heat Shock Sencar 6 | 1/5 | 0.563 |
| 10 | Heat Shock Sencar 7 | 1/5 | 0.718 |
| 10 | Heat Shock Sencar 8 | 1/5 | 0.548 |
| 10 | Heat Shock Sencar 9 | 1/5 | 0.718 |

In addition, the method of the present invention can be utilised to produce monoclonal antibodies. Indeed, animals having a serum like that of the heat-shocked Sencar Mouse 1 in Fig. 1 can be screened by competition experiments, and spleen cells can then be fused with SP2/0 lymphoma cells to obtain specific producer hybridomas. Under these conditions, these approaches can be used to study the immunological response of an animal to exposure to different proteins and their genetic variants, as for example virus proteins for vaccination, or fused proteins and cross-reaction of the recombinant variants. In addition it should be possible to study the hormonal or enzymatic activity of different proteins such as plasminogen activator, lymphokine, etc., as well as those of protein-engineered variants of such proteins. Animals carrying this type of tumor can be an excellent animal model to test in-vivo the activity, toxicology, oncogenicity etc. of new pharmaceutical molecules. In addition to the introduction of genes in tumor cells, a comparable system can be developed using the introduction of the described gene constructs, as example, into the genome of transgenic animals.

## Claims

1. A method for directly producing in an animal one or more substances resulting from the expression in suitable host-cells of recombinant genes driven by a stress-inducible promotor, which comprises the steps of:
   a) selecting as host-cells suitable cell lines capable of being transfected by vectors carrying recombinant DNA and thereafter proliferating in vitro or in vivo,
   b) making a gene expression construct by placing a gene coding for said substance(s) under the expression control of stress inducible promotor element in a suitably engineered DNA vector and incorporating said vector into said host-cells,
   c) inoculating selected warm blooded recipient animals with said transformed host-cells said animals being capable to foster said inoculated cells and allow said recombinant gene to be expressed therein,
   d) rearing and nursing the animals for a period of time sufficient for them to develop a response consecutive to inoculation,
   e) allowing or causing the host cells in the animal to be subjected to stress under conditions sufficient to induce the in-vivo expression of said synthetic gene but insufficient to harm the animal, whereby said gene substance is expressed and becomes present in substantial amounts in the body of the animal.

2. The method of claim 1, in which the promotor is a heat-shock promotor and the gene product is a secretable protein which, in step e), is secreted in the blood stream or the ascite fluid of the animal.

3. The method of claim 1 or 2, in which the expressed gene product produces an observable physiological response in the animal.

4. The method of claim 1, in which the animals are either not immunodepressed, or they are naturally or chemically immunodepressed.

5. The method of claim 4, in which the animals are not immunodepressed, whereby stress occurs due to reactions of rejection by the animal in response to inoculation.

6. The method of claim 1, in which inoculation is carried out subcutaneously (sc) or intraperitoneally (ip).

7. The method of claim 1, in which the animals develop temporary or permanent tumors in response to inoculation.

8. The method of claim 7, in which stress involves heating the animal, either entirely, or locally at the tumoral site.

9. The method of claim 1, in which the animal's response includes raising antibodies against said substances expressed by said recombinant genes, said antibodies being available by extraction from the body fluids of the animals.

10. The method of claim 9, in which the antibody is isolated by immunoprecipitation.

11. The method of claim 1, in which the coding sequence of the gene to be expressed is cellular genomic DNA, or synthetic DNA, e.g. cDNA.

12. The method of claim 1, in which said gene expresses products selected from hormones, enzymes, viral antigens, growth factors, etc...

13. The method of claim 1, in which the recipient animals are selected from mice, rats, rabbits, hamsters and the like.

14. The method of claim 9, in which said antibodies are isolated from the blood stream or the ascite fluids of the animals.

15. The method of claim 6, in which said host cells to be inoculated are in the form of strains grown in vitro or of fragments of tumors grown in nude mice.

16. The method of claim 1, in which the animals are treated before being inoculated to develop aseptic peritonitis.

17. The method of claim 1, in which the host cells are either naturally tumorigenic, or they are made so by transfection with an oncogen.

18. The method of claim 17, in which said host cells are selected from mouse fibroblasts, human amniotic cells, hamster ovary cells and the like.

19. The method of claim 4, in which the antibodies producing cells are removed from the animals, fused with strains of carcinoma cells to provide hybridoma cells and in which these cells are multiplied to produce monoclonal antibodies.

EP 0 336 523 A1

FIG. 1

FIG. 2

EP 0 336 523 A1

C
1  2  3  4

*FIG.5*

1  2  3  4  5

*FIG.3*

*FIG.4*

A
1  2  3  4

B
1  2  3  4

A

B
⊖ ⊕

1  2  3  4  5  6  7  8  9  10  11  12  13  14

51 52 53 54 55 56 (+)

*FIG.6*

13 13 14 14 15 15 16 16 (−)

41 42 43 71 72 74 (−)

31 31 32 32 33 33 34 34 (+)

*FIG.7*

EP 0 336 523 A1

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| D,Y | WO-A-8 700 201 (WHITEHEAD INSTITUTE) * Claims 26-34; page 7, line 23 - page 8, line 4 * | 1-4,6,7 ,11-13, 15,17, 18 | C 12 N 15/00 A 61 K 45/00 C 12 P 21/00 // (C 12 P 21/00 C 12 R 1:91 ) |
| Y | FR-A-2 487 851 (K.K. HAYASHIBARA SEIBUTSU KAGAKU KENKYUJO) * Page 1, line 18 - page 3, line 21; examples 1-4 * | 1-4,6,7 ,11-13, 15,17, 18 | |
| Y | GENE, vol. 49, no. 1, 1986, pages 1-8, Elsevier Science Publishers, Amsterdam, NL; M. DREANO et al.: "High-level heat-regulated synthesis of proteins in eukaryotic cells" * Whole article * | 1-4,6,7 ,11-13, 15,17, 18 | |
| O,Y | JOURNAL OF CELLULAR BIOCHEMISTRY, supplement no. 12B; UCLA SYMPOSIA ON GENE TRANSFER AND GENE THERAPY, 6th - 12th February 1988, page 186, abstract no. H148, Alan R. Liss Inc., New York, US; J.A. ZWIEBEL et al.: "In vivo growth hormone production by cells carrying recombinant rat growth hormone genes" * Abstract * | 1-4,6,7 ,11-13, 15,17, 18 | TECHNICAL FIELDS SEARCHED (Int. Cl.4) C 12 N |
| P,Y | EP-A-0 263 908 (BATTELLE MEMORIAL INSTITUTE) * Claims * | 1-4,6,7 ,11-13, 15,17, 18 | |

The present search report has been drawn up for all claims

| Place of search | Date of completion of the search | Examiner |
|---|---|---|
| THE HAGUE | 19-06-1989 | HUBER-MACK A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)